# EUROPÄISCHE PATENTANMELDUNG

(11) **EP 3 418 327 A1**
(43) Veröffentlichungstag der Anmeldung: **26.12.2018**
(21) Anmeldenummer: 17177164.5
(22) Anmeldetag: 21.06.2017
(51) Int. Cl.: C08J 9/02, B62D 29/00

(54) **EXPANDIERBARE ZUSAMMENSETZUNG MIT EINEM CYCLISCHEN CARBONAT UND EINER BASE**

(71) Anmelder: Henkel AG & Co. KGaA, 40589 Düsseldorf (DE)
(72) Erfinder: PÖLLER, Sascha, 40589 Düsseldorf (DE); KOHLSTRUNG, Rainer, 68723 Plankstadt (DE); RONGIER, Thomas, 69590 Saint Symphorien Sur Coise (FR)

(57) **Zusammenfassung**

Die vorliegende Anmeldung betrifft eine thermisch expandierbare Zusammensetzung, die mindestens eine organische Verbindung umfassend mindestens zwei cyclische Carbonatgruppen, mindestens eine Verbindung mit mindestens einer funktionalen Gruppe mit einem pKs-Wert von größer 3 und mindestens eine Base umfasst, Formkörper, die diese Zusammensetzung enthalten, sowie ein Verfahren zum Abdichten und Füllen von Hohlräumen in Bauteilen, zum Verstärken oder Versteifen von Bauteilen, insbesondere hohlen Bauteilen, und zum Verkleben von beweglichen Bauteilen unter Verwendung derartiger Formkörper.

## Beschreibung

Die vorliegende Anmeldung betrifft eine thermisch expandierbare Zusammensetzung, die mindestens eine organische Verbindung umfassend mindestens zwei cyclische Carbonatgruppen, mindestens eine Verbindung mit mindestens einer funktionalen Gruppen mit einem pKs-Wert von größer 3 und mindestens eine Base umfasst, Formkörper, die diese Zusammensetzung enthalten, sowie ein Verfahren zum Abdichten und Füllen von Hohlräumen in Bauteilen, zum Verstärken oder Versteifen von Bauteilen, insbesondere hohlen Bauteilen, und zum Verkleben von beweglichen Bauteilen unter Verwendung derartiger Formkörper.

Moderne Fahrzeuge und Fahrzeugteile weisen eine Vielzahl von Hohlräumen auf, die abgedichtet werden müssen, um den Eintritt von Feuchtigkeit und Verschmutzungen zu verhindern, da diese zur Korrosion an den entsprechenden Karosserieteilen von innen heraus führen kann. Dies trifft insbesondere auf moderne selbst tragende Karosseriekonstruktionen zu, bei denen eine schwere Rahmenkonstruktion durch leichtgewichtige, strukturfeste Rahmengerüste aus vorgefertigten Hohlraumprofilen ersetzt wird. Derartige Konstruktionen weisen systembedingt eine Reihe von Hohlräumen auf, die gegen das Eindringen von Feuchtigkeit und Verschmutzungen abgedichtet werden müssen. Derartige Abdichtungen dienen weiterhin dem Zweck, die Weiterleitung von Luftschall in derartigen Hohlräumen zu vermeiden und somit unangenehme Fahrzeuglauf- und Windgeräusche zu mindern und somit den Fahrkomfort im Fahrzeug zu steigern.

Schottteile, die eine abdichtende und/oder akustische Wirkung in derartigen Hohlräumen bewirken, werden häufig als "pillar filler", "baffles" oder "acoustic baffles" bezeichnet. Sie bestehen in der Regel entweder vollständig aus thermisch expandierbaren Formkörpern oder aus Formkörpern, die einen Träger und in ihrem Peripheriebereich expandierbare polymere Zusammensetzungen enthalten. Diese Schottteile werden im Rohbau durch Einhängen, Einclipsen, Verschrauben oder Anschweißen an den offenen Baustrukturen befestigt. Nach dem Schließen der Baustrukturen im Rohbau und den weiteren Vorbehandlungen der Karosserie wird dann die Prozesswärme der Öfen zur Härtung der kathodischen Tauchlackierung ausgenutzt, um die Expansion des expandierbaren Teils des Schottteils auszulösen um somit den Querschnitt des Hohlraums abzudichten.

In modernen Fahrzeugen werden ferner immer häufiger metallische Leichtbauteile für eine konstant maßhaltige Serienfertigung mit vorgegebener Steifigkeit und Strukturfestigkeit benötigt. Insbesondere im Fahrzeugbau ist im Zuge der gewünschten Gewichtsersparnis Bedarf für metallische Leichtbauteile aus dünnwandigen Blechen, die trotzdem ausreichende Steifigkeit und Strukturfestigkeit besitzen. Auch hier kommen Formkörper aus thermisch expandierbaren Zusammensetzungen, die die nötigen Stützeigenschaften verleihen zum Einsatz.

Entsprechende thermisch expandierbare Zusammensetzung werden zum Beispiel in den Schriften WO 2008/034755, WO 2007/039309, WO 2013/017536 und der deutschen Anmeldung 10 2012 221 192.6 beschrieben. Diese thermisch expandierbaren Zusammensetzungen finden auch Einsatz im Automobilbereich.

In derartigen expandierbaren Zusammensetzungen, wie beispielsweise Kautschuk-Vulkanisaten (Schwefel, Peroxid oder Benzochinondioxim) zur Abdichtung und Verklebung, Ethylenvinylacetatbasierten Hohlraumabschottungen, Epoxid-basierten Stützschäumen und expandierbaren Versiegelungsmassen im Automobilbau, werden heutzutage Treibmittel, wie beispielsweise ADCA (Azodicarbonamid), OBSH (4,4'-Oxybis(benzolsulfonylhydrazid)), DNPT (Dinitrosopentamethylentetramin), PTSS (p-Toluolsemicarbazid), BSH (Benzol-4-sulfonohydrazid), TSH (Toluol-4-sulfonohydrazid), 5-PT (5-Phenyltetrazol) und ähnliche, verwendet.

Diese Treibmittel haben den Nachteil, dass sie eine respiratorische Sensitivierung auslösen können, aus toxikologischer Sicht generell bedenklich sind, oder explosiv sind. Darüber hinaus entstehen bei ihrem Zerfall Nebenprodukte wie Ammoniak, Formamid, Formaldehyd oder Nitrosamine, die gemäß der Global Automotive Declarable Substance List (GADSL), IFA-KMR-List 08/2012 oder dem BGIA-Bericht "Index of hazardous substances 2012" im Automobilbau verboten sind. Zusätzlich ist bei der Verwendung von Treibmitteln der VOC-Gehalt (Gehalt flüchtiger organischer Verbindungen) sehr hoch. Daher wurden diese Treibmittel von der REACH-Verordnung als SVHCs eingestuft und werden mit großer Wahrscheinlichkeit verboten werden. Als Alternative zu diesen Treibmitteln können physikalische Treibmittel verwendet werden, die üblicherweise aus einem von einer Polymerhülle eingekapselten Gas oder leicht flüchtige Substanzen, wie Pentan, bestehen. Die Anwendung dieser Treibmittel ist jedoch nachteilig, da verhältnismäßig viel Treibmittelmasse eingesetzt werden muss, um hohe Expansionsraten zu erzielen. Ferner dürfen die expandierbaren Zusammensetzungen keine Bestandteile enthalten, die die Stabilität der Polymerhülle beeinflusst, da sonst Treibmittel vor dem zweckgemäßen Einsatz verdampft, welches zudem häufig leicht entzündlich ist.

Aufgabe der vorliegenden Erfindung war es daher, thermisch expandierbare Zusammensetzungen zur Verfügung zu stellen, welche vorzugsweise lagerstabil bei Raumtemperatur sind und Treibmittel enthalten, die weder explosiv noch toxisch bedenklich sind und ferner gute Expansionsraten aufweisen. Zudem sollten die Edukte möglichst gut in den resultierenden Schäumen eingebunden werden.

Diese Aufgabe wird überraschender Weise durch thermisch expandierbare Zusammensetzungen gelöst, die
(1) mindestens eine organische Verbindung umfassend mindestens zwei cyclische Carbonatgruppen,
(2) mindestens eine Verbindung mit mindestens einer funktionalen Gruppen mit einem pKs-Wert von größer 3, und
(3) mindestens eine Base umfassen.

Entsprechende Zusammensetzungen überwinden die bekannten Nachteile und weisen gleichzeitig einehervorragende Expansion auf. Dabei fungiert die mindestens eine organische Verbindung umfassend mindestens zwei cyclische Carbonatgruppen sowohl als Bindemittel als auch als Treibmittel. Dadurch, dass das Treibmittel gleichzeitig das Bindemittel darstellt, kann eine sehr hohe und gleichmäßige Expansion erreicht werden. Zudem wird hierdurch das Treibmittel ideal in das resultierende Bindemittelgerüst eingebaut, da das Treibmittel selber das Bindemittel darstellt, und es verbleiben keine ungewollten flüchtigen Reste, die toxisch sein könnten.

Daher betrifft die vorliegende Erfindung in einem ersten Aspekt eine thermisch expandierbare Zusammensetzung, enthaltend
(1) mindestens eine organische Verbindung umfassend mindestens zwei cyclische Carbonatgruppen;
(2) mindestens eine Verbindung mit mindestens einer funktionalen Gruppen mit einem pKs-Wert von größer 3; und
(3) mindestens eine Base.

In einem weiteren Aspekt betrifft die Erfindung einen Formkörper, der eine erfindungsgemäße thermisch expandierbare Zusammensetzung aufweist.

Des Weiteren richtet sich die vorliegende Erfindung auf ein Verfahren zum Abdichten und Füllen von Hohlräumen in Bauteilen, zum Verstärken oder Versteifen von Bauteilen, insbesondere hohlen Bauteilen, und zum Verkleben von beweglichen Bauteilen unter Verwendung einer hierin offenbarten thermisch expandierbaren Zusammensetzung oder Formkörper. Dabei wird das Aufschäumen der expandierbaren Zusammensetzung durch die mindestens eine organische Verbindung umfassend mindestens eine cyclische Carbonatgruppe hervorgerufen, vorzugsweise erfolgt eine Expansion der expandierbaren Zusammensetzung von mehr als 5 Vol.-%, vorzugsweise mehr als 50 Vol.-%, insbesondere mehr als 100 Vol.-%, jeweils bezogen auf die expandierbare Zusammensetzung vor der Expansion.

In einem noch anderen Aspekt betrifft die vorliegende Erfindung die Verwendung eines hierin beschriebenen Formkörpers zur akustischen Abdichtung von Hohlräumen in Bauteilen und/oder zur Abdichtung von Hohlräumen in Bauteilen gegen Wasser und/oder Feuchtigkeit oder zum Verstärken oder Versteifen von Bauteilen, insbesondere hohlen Bauteilen.

"Mindestens ein", wie hierin verwendet, bezieht sich auf 1 oder mehr, beispielsweise 1, 2, 3, 4, 5, 6, 7, 8, 9 oder mehr. Im Zusammenhang mit Bestandteilen der hierin beschriebenen KatalysatorZusammensetzungen bezieht sich diese Angabe nicht auf die absolute Menge an Molekülen sondern auf die Art des Bestandteils. "Mindestens ein organisches Carbonat" bedeutet daher beispielsweise ein oder mehrere organische Carbonate, d.h. eine oder mehrere verschiedene Arten von organischen Carbonaten. Zusammen mit Mengenangaben beziehen sich die Mengenangaben auf die Gesamtmenge der entsprechend bezeichneten Art von Bestandteil, wie bereits oben definiert.

"Ganze Zahl", wie hierin verwendet, ist 0 und alle natürliche Zahlen. "Natürliche Zahl", wie hierin verwendet, bezieht sich auf die allgemeingültige Bedeutung des Begriffs und ist 1, 2, 3, 4, 5, 6, 7, 8, 9, 10 und mehr.

Als einen wesentlichen Bestandteil enthalten die Zusammensetzungen mindestens eine organische Verbindung umfassend mindestens zwei cyclische Carbonatgruppen, welche den Vorteil aufweist, dass sie weder gesundheitsschädlich noch explosiv ist und bei der Expansion keine flüchtigen organischen Verbindungen (VOC) entstehen. Die Zersetzungsprodukte sind im wesentlichen CO₂ und das organische "Rest-Molekül", an welches die cyclische Carbonatgruppe gebunden war. Das organische "Rest-Molekül" bildet dann das Bindemittelsystem, so dass keine flüchtigen organischen Verbindungen freigesetzt werden. Ferner weisen die damit hergestellten Produkte eine gleichmäßige Schaumstruktur über den gesamten Prozesstemperaturbereich auf, der für die Härtung genutzt wird.

Dabei fungiert die mindestens eine organische Verbindung umfassend mindestens zwei cyclische Carbonatgruppe sowohl als Bindemittel als auch als Treibmittel. Unter einem chemischen Treibmittel werden erfindungsgemäß Verbindungen verstanden, die sich bei Einwirkung von Wärme zersetzen und dabei Gase freisetzen. Die mindestens eine organische Verbindung weist mindestens zwei cyclische Carbonatgruppen auf, die an die organische Verbindung gebunden ist. Die cyclischen Carbonatgruppen sind zwingend an eine organische Verbindung gebunden. Eine Verbindung, die lediglich aus einer cyclischen Carbonatgruppe besteht, wie Ethylencarbonat, stellt keine erfindungsgemäße Verbindung dar. Rein cyclische Carbonate als Treibmittel, wie Ethylencarbonat, hinterlassen beim Zersetzten flüchtige organische Verbindungen, die nicht oder nur schlecht in der geschäumten Zusammensetzung eingebunden werden könnten und zudem nicht als Bindemittel dienen können.

Vorzugsweise weist die organische Verbindung mindestens zwei bis vier cyclische Carbonatgruppen auf. Bevorzugt ist die organische Verbindung ein Polyether und/oder Polyester, insbesondere Polyether, an welchen mindestens eine, vorzugsweise mindestens zwei cyclische Carbonatgruppen gebunden sind.

In einer weiteren bevorzugten Ausführungsform ist die organische Verbindung umfassend mindestens zwei cyclische Carbonatgruppen eine Verbindung der Formel (I)

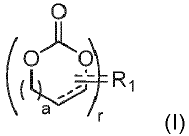

Hierbei ist 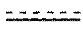 eine Einfach- oder Doppelbindung, vorzugsweise eine Einfachbindung. Es ist selbstverständlich, dass wenn der Ring eine Doppelbindung enthält, R₁ nicht über eine exo-Doppelbindung sondern über eine Einfachbindung gebunden ist und umgekehrt. R₁ ist lineares oder verzweigtes, substituiertes oder unsubstituiertes Alkyl, lineares oder verzweigtes, substituiertes oder unsubstituiertes Heteroalkyl, lineares oder verzweigtes, substituiertes oder unsubstituiertes Alkenyl, lineares oder verzweigtes, substituiertes oder unsubstituiertes Alkinyl, substituiertes oder unsubstituiertes Cycloalkyl, substituiertes oder unsubstituiertes Aryl, oder -C(O)-R^{a}, wobei R^{a} H, lineares oder verzweigtes, substituiertes oder unsubstituiertes Alkyl, lineares oder verzweigtes, substituiertes oder unsubstituiertes Heteroalkyl, lineares oder verzweigtes, substituiertes oder unsubstituiertes Alkenyl, lineares oder verzweigtes, substituiertes oder unsubstituiertes Alkinyl, substituiertes oder unsubstituiertes Cycloalkyl, substituiertes oder unsubstituiertes Aryl ist, vorzugsweise ein lineares oder verzweigtes, substituiertes oder unsubstituiertes Heteroalkyl, wobei R₂ vorzugsweise mindestens 2, vorzugsweise mindestens 4 Kohlenstoffatome umfasst. In der Formel (II) ist a eine ganze Zahl von 0 bis 5, vorzugsweise 0 oder 1 und besonders bevorzugt 0. Ferner ist r eine natürliche Zahl von 2 bis 10, vorzugsweise von 2 bis 4.

"Substituiert", wie im Zusammenhang mit den Alkyl- oder Heteroalkylresten hierin verwendet, bedeutet, dass die entsprechende Gruppe mit einem oder mehreren Substituenten ausgewählt aus der Gruppe bestehend aus -OR', -COOR', -NR'R", -C(=X)NR'R", -NR"C(=X)NR'R", Halogen, unsubstituiertem C₆₋₁₄ Aryl, unsubstituiertem C₂₋₁₄ Heteroaryl enthaltend 1 bis 5 Heteroatome ausgewählt aus O, N und S, unsubstituiertem C₃₋₁₀ Cycloalkyl und unsubstituiertem C₂₋₁₀ Heteroalicyclyl enthaltend 1 bis 5 Heteroatome ausgewählt aus O, N und S substituiert ist.

"Substituiert", wie im Zusammenhang mit den Aryl- und Cycloalkylresten hierin verwendet, bedeutet, dass die entsprechende Gruppe mit einem oder mehreren Substituenten ausgewählt aus der Gruppe bestehend aus -OR', -COOR', -NR'R", -C(=X)NR'R", -NR"C(=X)NR'R", Halogen, unsubstituiertem C₁₋₁₀ Alkyl und -CH₂-Aryl substituiert sein kann, wobei die Arylgruppe in der -CH₂-Aryl-Gruppe wiederum mit-OR', -COOR', -NR'R", -C(=X)NR'R", -NR"C(=X)NR'R", Halogen und unsubstituiertem C₁₋₁₀ Alkyl substituiert sein kann.

R' und R" sind dabei unabhängig voneinander ausgewählt aus H, unsubstituiertem C₁₋₁₀ Alkyl, unsubstituiertem C₆₋₁₄ Aryl, unsubstituiertem C₂₋₁₄ Heteroaryl, unsubstituiertem C₃₋₁₀ Cycloalkyl, unsubstituiertem C₂₋₁₀ Heteroalicyclyl, Alkylaryl, Arylalkyl, Heteroarylalkyl und Alkylheteroaryl.

"Alkyl", wie hierin verwendet, bezieht sich auf lineare oder verzweigte Alkylgruppen vorzugsweise mit 1 bis 30 Kohlenstoffatomen, wie beispielsweise Methyl, Ethyl, n-Propyl, iso-Propyl, n-Butyl, n-Pentyl, n-Hexyl, n-Heptyl, n-Octyl, n-Nonyl, n-Decyl, n-Undecyl und n-Dodecyl sowie die linearen C₁₄, C₁₆ und C₁₈ Alkylreste. In verschiedenen Ausführungsformen sind die Alkylreste kurzkettige C₁₋₄ Alkylreste, insbesondere unsubstituierte, lineare C₁₋₄ Alkylreste. Die Alkylreste können substituiert oder unsubstituiert sein, sind aber vorzugsweise unsubstituiert. Wenn sie substituiert sind, werden die Substituenten insbesondere ausgewählt aus der oben beschriebenen Gruppe von Substituenten.

"Heteroalkyl", wie hierin verwendet, bezieht sich auf Alkylreste wie oben definiert, in denen mindestens ein Kohlenstoffatom durch ein Heteroatom ersetzt ist, insbesondere N oder O, besonders bevorzugt O.

"Aryl", wie hierin verwendet, bezieht sich auf aromatische Gruppen mit 5 bis 30 Kohlenstoffatomen, die mindestens einen aromatischen Ring, aber auch mehrere kondensierte Ringe aufweisen können, wie beispielsweise Phenyl, Naphthyl, Anthracenyl und dergleichen. Die Arylreste können substituiert oder unsubstituiert sein. Wenn sie substituiert sind, werden die Substituenten ausgewählt aus der oben beschriebenen Gruppe.

"Heteroaryl", wie hierin verwendet, bezieht sich auf Arylreste wie oben definiert, in denen mindestens ein Kohlenstoffatom durch ein Heteroatom ersetzt ist, insbesondere N, S oder O, besonders bevorzugt O.

"Halogen", wie hierin verwendet, bezieht sich auf F, Cl, Br und I.

"Cycloalkyl", wie hierin verwendet, bezieht sich auf nicht-aromatische, cyclische Kohlenwasserstoffe, insbesondere cyclische Alkyl- oder Alkenylreste wie oben definiert, z.B. Cyclopentyl-, Cyclohexyl- und Cyclohexenyl-Reste. Wenn sie substituiert sind, werden die Substituenten ausgewählt aus der oben beschriebenen Gruppe.

"Heteroalicyclyl", wie hierin verwendet, bezieht sich auf Cycloalkylreste wie oben definiert, in denen mindestens ein Kohlenstoffatom durch ein Heteroatom ersetzt ist, insbesondere N, S oder O, besonders bevorzugt O.

In einer anderen bevorzugten Ausführungsform ist die organische Verbindung umfassend mindestens zwei cyclische Carbonatgruppen eine Verbindung der Formel (II)

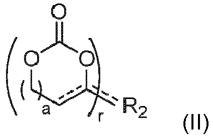

In der Formel (II) sind 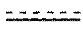 , a und r wie oben definiert. Ferner ist R₂ wie R₁ definiert. Auch hier ist es selbstverständlich, dass wenn der Ring eine Doppelbindung enthält, R₂ nicht über eine exo-Doppelbindung sondern über eine Einfachbindung gebunden ist und umgekehrt. R₂ ist ferner wie R₁ definiert und vorzugsweise ein lineares oder verzweigtes, substituiertes oder unsubstituiertes Heteroalkyl.

Besonders bevorzugt sind solche Verbindungen, in denen R₁ bzw. R₂ eine zwei- oder mehrwertige Heteroalkylgruppe, wie beispielsweise ein Pentaerithrytolrest, sind, an welchen dann 2 oder mehr der cyclischen Carbonatgruppen gebunden sind. Insbesondere stehen R₁ bzw. R₂ für Polyester und Polyether, bevorzugt Polyether, an welche dann 2 oder mehr der cyclischen Carbonatgruppen gebunden sind.

Zur Herstellung von Alkenyletherpolyole werden häufig cyclische Carbonate eingesetzt, die Derivate der Carbonate der Formeln (I) und (II) entsprechen. Beispielhafte Derivate davon schließen solche ein, die an den Ring-Methylengruppen, insbesondere denen die nicht den R₁ bzw. R₂ Rest tragen, substituiert sind, beispielsweise mit organischen Resten, insbesondere linearen oder verzweigten, substituierten oder unsubstituierten Alkyl- oder Alkenylresten mit bis zu 20 Kohlenstoffatomen, insbesondere =CH₂ und -CH=CH₂, oder linearen oder verzweigten, substituierten oder unsubstituierten Heteroalkyl- oder Heteroalkenylresten mit bis zu 20 Kohlenstoffatomen und mindestens einem Sauerstoff- oder Stickstoffatom, oder funktionellen Gruppen, wie beispielsweise -OH oder-COOH. Beispiele für solche Derivate schließen beispielsweise 4-Methylen-1,3-Dioxolan-2-on, das an der 5-Position den R₁ bzw. R₂ Rest trägt, oder di-(Trimethylolpropan)dicarbonat, wobei der R₁ bzw. R₂ Rest in der 5-Position ein Methylen-Trimethylolmonocarbonatrest ist.

In verschiedenen Ausführungsformen in denen der R₁ bzw. R₂ Rest über eine Einfachbindung gebunden ist, kann das Ring-Kohlenstoffatom, dass den R₁ bzw. R₂ Rest trägt mit einem weiteren Substituenten, der wie die oben genannten Substituenten für die anderen Ring-Methylengruppe definiert ist, substituiert sein.

Weitere Derivate sind solche in denen eines oder beide der Ring-Sauerstoffatome durch Schwefelatome ersetzt sind sowie solche in denen alternativ oder zusätzlich das Carbonyl-Sauerstoffatom durch ein Schwefelatom ersetzt ist.

Bevorzugt werden die organischen Verbindungen umfassend mindestens zwei cyclische Carbonatgruppen hergestellt durch Umsetzung eines Epoxides oder eines Oxetans mit Kohlendioxid, insbesondere die Verbindungen der Formel (II) werden so hergestellt. Bei der Umsetzung eines Epoxides wird eine Verbindung mit einem fünfgliedrigen cyclischen Carbonat erhalten, während bei der Umsetzung eines Oxetans eine Verbindung mit einem sechsgliedrigen cyclischen Carbonat erhalten wird. Besonders bevorzugt werden die eingesetzten organischen Verbindungen umfassend mindestens zwei cyclische Carbonatgruppen hergestellt durch Umsetzung eines Epoxides, vorzugsweise eines Polyepoxides, insbesondere die später genannten Polyepoxide, mit Kohlendioxid. Besonders bevorzugt sind Epoxide basierend auf einem mehrwertigen aliphatischen Polyether, wie Ether des Pentaerithrytols, oder aromatische Epoxide, insbesondere Epoxide basieren auf Bisphenol-Derivaten. Bei der Umsetzung eines Polyepoxides (Verbindung mit mindestens zwei, insbesondere genau zwei Epoxidgruppen pro Molekül) mit Kohlendioxids muss die Umsetzung nicht zwangsläufig vollständig erfolgen, vorzugsweise erfolgt eine Umsetzung von 70 bis 100% der Epoxidgruppen. Allerdings ist eine vollständige Umsetzung bevorzugt.

In einer noch weiteren bevorzugten Ausführungsform der vorliegenden Erfindung ist die organische Verbindung umfassend mindestens zwei cyclische Carbonatgruppen eine Verbindung der Formel (III) oder (IV).

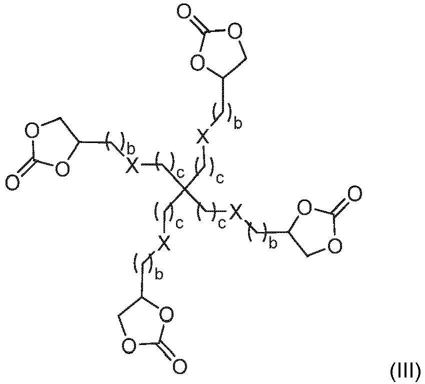

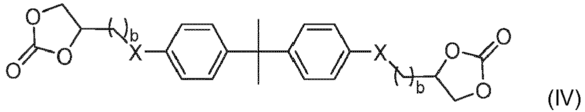

Hierbei ist jedes b und jedes c jeweils unabhängig voneinander eine natürliche Zahl von 1 bis 5 vorzugsweise 1 oder 2 und besonders bevorzugt 1. Jedes b kann genauso wie jedes c verschieden oder gleich sein. Vorzugsweise ist jedes b und jedes c gleich. Ferner ist jedes X unabhängig ausgewählt aus der Gruppe bestehend aus O und S. Vorzugsweise ist jedes X O.

In einer noch weiteren bevorzugten Ausführungsform ist die organische Verbindung umfassend mindestens eine cyclische Carbonatgruppe eine Verbindung der Formel (V) oder (VII)

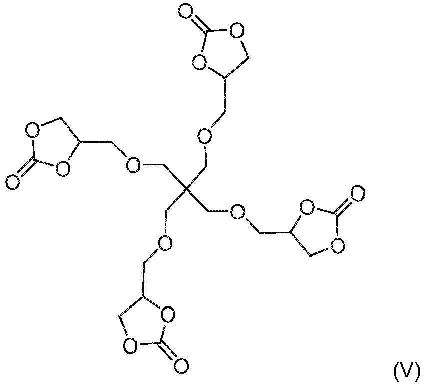

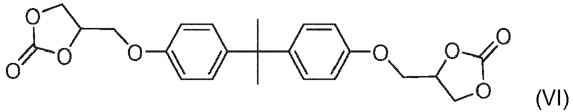

Die erfindungsgemäßen organischen Carbonate sind synthetisch über ein entsprechendes Epoxid und CO₂ als Ausgangsstoffe in Anwesenheit eines Katalysators unter milden Bedingungen zugänglich.

Die thermisch expandierbaren Zusammensetzungen enthalten die organischen Verbindungen umfassend mindestens zwei cyclische Carbonatgruppen vorzugsweise in einer Menge von mindestens 20 Gew.-%, insbesondere mindestens 25 Gew.-%, bzw. vorzugsweise in einer Menge von 20 bis 90 Gew.-%, vorzugsweise 25 bis 80 Gew.-%, besonders bevorzugt 25 bis 75 Gew.-%, noch weiter bevorzugt 25 bis 70 Gew.-% und am bevorzugtesten 30 bis 65 Gew.-%. Dabei beziehen sich die Gew.-%, soweit nicht anders beschrieben, auf das Gesamtgewicht der Gesamtzusammensetzung vor der Expansion.

In einer anderen bevorzugten Ausführungsform enthalten die thermisch expandierbaren Zusammensetzungen die als Treibmittel und Bindemittel eingesetzten organischen Verbindungen umfassend mindestens zwei cyclische Carbonatgruppen vorzugsweise in einer Menge von mindestens 20 Gew.-%, insbesondere mindestens 25 Gew.-%, bzw. vorzugsweise in einer Menge von 20 bis 90 Gew.-%, vorzugsweise 25 bis 80 Gew.-%, besonders bevorzugt 25 bis 75 Gew.-%, noch weiter bevorzugt 25 bis 70 Gew.-% und am bevorzugtesten 30 bis 65 Gew.-%, jeweils bezogen auf das Bindemittelsystem (Gesamtgewicht der Gesamtzusammensetzung vor der Expansion ohne Füllstoffe).

Optional können der erfindungsgemäßen Zusammensetzung weitere chemische Treibmittel zugesetzt werden.

Bevorzugt sind die expandierbaren Zusammensetzungen frei von ADCA (Azodicarbonamid) und OBSH (4,4'-Oxybis(benzolsulfonylhydrazid), insbesondere frei von ADCA (Azodicarbonamid), OBSH (4,4'-Oxybis(benzolsulfonylhydrazid)), DNPT (Dinitrosopentamethylentetramin), PTSS (p-Toluolsemicarbazid), BSH (Benzol-4-sulfonohydrazid), TSH (Toluol-4-sulfonohydrazid) und 5-PT (5-Phenyltetrazol), besonders bevorzugt frei von anderen exothermen Treibmitteln, insbesondere frei von anderen Treibmittel. Bevorzugt sind die expandierbaren Zusammensetzungen frei von anorganischen Carbonaten. "Frei von", wie in diesem Zusammenhang verwendet, bedeutet, dass die Menge des entsprechenden Stoffs in dem Reaktionsgemisch weniger als 0,05 Gew.-%, vorzugsweise weniger als 0,01 Gew.-%, noch bevorzugter weniger als 0,001 Gew.-%, bezogen auf das Gesamtgewicht des Reaktionsgemischs beträgt, insbesondere gänzlich frei.

Allgemein können die expandierbaren Zusammensetzungen neben der beschriebenen mindestens einen organischen Verbindung umfassend mindestens zwei cyclische Carbonatgruppe noch weitere Bindemittel enthalten. In einer bevorzugten Ausführungsform enthält die Zusammensetzung noch mindestens ein weiteres reaktives Bindemittel, insbesondere in Kombination mit mindestens einen Härter und/oder Beschleuniger. Bevorzugt wird das weitere reaktive Bindemittel ausgewählt aus der Gruppe von Epoxiden, Kautschuken und peroxidisch vernetzbaren Polymeren, insbesondere Epoxiden.

Ein bevorzugter Gegenstand enthält als weiteres reaktives Bindemittel Epoxide. Als Epoxidharze eignen sich eine Vielzahl von Polyepoxiden, die mindestens 2 1,2- Epoxygruppen pro Molekül haben. Das Epoxid-Äquivalent dieser Polyepoxide kann zwischen 150 und 50000, vorzugsweise zwischen 170 und 5000, varüeren. Die Polyepoxide können grundsätzlich gesättigte, ungesättigte, cyclische oder acyclische, aliphatische, alicyclische, aromatische oder heterocyclische Polyepoxidverbindungen sein. Beispiele für geeignete Polyepoxide schliessen die Polyglycidylether ein, die durch Reaktion von Epichlorhydrin oder Epibromhydrin mit einem Polyphenol in Gegenwart von Alkali hergestellt werden. Hierfür geeignete Polyphenole sind beispielsweise Resorcin, Brenzkatechin, Hydrochinon, Bisphenol A (Bis-(4-Hydroxy-phenyl)-2,2-propan)), Bisphenol F (Bis(4-hydroxyphenyl)methan), Bis(4-hydroxyphenyl)-1,1-isobutan, 4,4'-Dihydroxybenzophenon, Bis(4-hydroxyphenyl)-1,1-ethan, 1,5-Hydroxynaphthalin. Weitere geeignete Polyphenole als Basis für die Polyglycidylether sind die bekannten Kondensationsprodukte aus Phenol und Formaldehyd oder Acetaldehyd vom Typ der Novolakharze.

Weitere prinzipiell geeignete Polyepoxide sind die Polyglycidylether von Polyalkoholen oder Diaminen. Diese Polyglycidylether leiten sich von Polyalkoholen wie Ethylenglykol, Diethylenglykol, Triethylenglykol, 1,2-Propylenglykol, 1,4-Butylenglykol, Triethylenglykol, 1,5-Pentandiol, 1,6-Hexandiol oder Trimethylolpropan ab.

Weitere Polyepoxide sind Polyglycidylester von Polycarbonsäuren, beispielsweise Umsetzungen von Glycidol oder Epichlorhydrin mit aliphatischen oder aromatischen Polycarbonsäuren wie Oxalsäure, Bernsteinsäure, Glutarsäure, Terephthalsäure oder Dimerfettsäure.

Weitere Epoxide leiten sich von den Epoxidierungsprodukten olefinisch ungesättigter cycloaliphatischer Verbindungen oder von nativen Ölen und Fetten ab.

Je nach Anwendungszweck kann es bevorzugt sein, dass die Zusammensetzung zusätzlich ein flexibilisierend wirkendes Harz enthält. Hierbei kann es sich ebenfalls um ein Epoxidharz handeln. Als flexibilisierend wirkende Epoxyharze können die an sich bekannten Addukte aus Carboxylterminierten Butadienacrylnitrilcopolymeren (CTBN) und flüssigen Epoxidharzen auf der Basis des Diglycidylethers vom Bisphenol A eingesetzt werden. Konkrete Beispiele sind die Umsetzungsprodukte Hycar CTBN 1300 X8, 1300 X13 oder 1300 X15 der Firma B. F. Goodrich mit flüssigen Epoxidharzen. Weiterhin lassen sich auch die Umsetzungsprodukte von aminoterminierten Polyalkylenglykolen (Jeffamine) mit einem Überschuss an flüssigen Polyepoxiden einsetzen. Grundsätzlich können auch Umsetzungsprodukte von Mercapto-funktionellen Prepolymeren oder flüssige Thiol-Polymere mit einem Überschuss an Polyepoxiden als flexibilisierende Epoxidharze erfindungsgemäß eingesetzt werden. Ganz besonders bevorzugt sind jedoch die Umsetzungsprodukte von polymeren Fettsäuren, insbesondere der Dimerfettsäure mit Epichlorhydrin, Glycidol oder insbesondere Diglycidylether des Bisphenols A (DGBA).

Die thermisch expandierbaren Zubereitungen enthalten vorzugsweise mindestens 2 Gew.-% mindestens eines weiteren Epoxides. Thermisch expandierbare Zubereitungen, die 2 bis 50 Gew.-%, insbesondere 5 bis 30 Gew.-% mindestens eines Epoxides, jeweils bezogen auf die Gesamtmasse der thermisch expandierbaren Zubereitung, enthalten, sind besonders bevorzugt.

Als thermisch aktivierbare oder latente Härter für das Epoxidharz-Bindemittelsystem aus den genannten Komponenten können Guanidine, substituierte Guanidine, Melaminharze, Guanamin-Derivate, cyclische tertiäre Amine, aromatische Amine und/oder deren Mischungen eingesetzt werden. Dabei können die Härter stöchiometrisch mit in die Härtungsreaktion einbezogen sein. Sie können jedoch auch katalytisch wirksam sein. Beispiele für substituierte Guanidine sind Methylguanidin, Dimethylguanidin, Trimethylguanidin, Tetramethylguanidin, Methylisobiguanidin, Dimethylisobiguanidin, Tetramethylisobiguanidin, Hexamethylisobiguanidin, Hepamethylisobiguanidin und ganz besonders Cyanoguanidin (Dicyandiamid). Als Vertreter für geeignete Guanamin-Derivate seien alkylierte Benzoguanamin-Harze, Benzoguanamin-Harze oder Methoximethyl-ethoxymethylbenzoguanamin genannt. Für einkomponentige, hitzehärtende Formkörper ist das Auswahlkriterium die niedrige Löslichkeit dieser Stoffe bei Raumtemperatur in dem Harzsystem, so dass hier feste, feinvermahlene Härter den Vorzug haben. Insbesondere ist Dicyandiamid geeignet. Damit ist eine gute Lagerstabilität der hitzehärtbaren Formkörper gewährleistet.

Vorzugsweise liegt der Härter und/oder Beschleuniger in einer Menge von insgesamt mindestens 0,25 Gew.-% und insbesondere von mindestens 1,5 Gew.-% bezogen auf die Gesamtzusammensetzung vor der Expansion. Mehr als insgesamt 5 Gew.-% bezogen auf die Gesamtmasse der Zusammensetzung sind jedoch in der Regel nicht erforderlich

Zur Verbesserung der Stoßfestigkeit können ferner ein oder mehrere so genannte "impact modifier" anwesend sein, wie sie im Stand der Technik für diesen Zweck bekannt sind. Beispiele sind thermoplastische Harze, die vorzugsweise gegenüber Epoxidgruppen reaktive Gruppen tragen. Weiterhin sind natürliche oder synthetische Kautschuke für diesen Zweck geeignet. Konkrete Beispiele hierfür können dem Dokument WO 2007/004184 in den Abschnitten [27] und [28] (Seiten 6 und 7) entnommen werden.

Als weiteren Bestandteil umfasst die thermisch expandierbare Zusammensetzung mindestens eine Verbindung mit mindestens einer funktionalen Gruppen, insbesondere mindestens zwei funktionalen Gruppen, mit einem pKs-Wert von größer 3, vorzugsweise größer 5, insbesondere größer 7, bevorzugt größer 9. Beispielsweise könnte diese Verbindung ein Novolak (Phenolharz) sein, welche als funktionale Gruppen aromatische OH-Gruppen aufweist, wobei reines Phenol einen pKs-Wert von ungefähr 10 aufweist. Vorzugsweise weist die mindestens eine funktionale Gruppe einen pKs-Wert von kleiner 30, insbesondere kleiner 25, bevorzugt kleiner 20 auf.

Die Verbindung mit mindestens einer funktionalen Gruppen wird typischerweise von der Base deprotoniert und wirkt als schwaches Nucleophil, welches die Ringöffnung der Carbonate und damit die Freisetzung von CO₂ ermöglicht. Zudem können die funktionalen Gruppen mit dem Carbonat reagieren und so das Bindemittelsystem aufbauen. Zu starke Nucleophile hingegen führen in der Regel dazu, dass die Freisetzung des Kohlendioxids nicht auftritt. Diese Verbindung sollte bevorzugt keine funktionale Gruppe mit einem pKs-Wert von kleiner 3 aufweisen bzw. keinen Wasserstoff mit einem pKs-Wert von kleiner 3 aufweisen.

Bevorzugt enthält die expandierbare Zusammensetzung eine Verbindung mindestens einer, bevorzugt mindestens zwei funktionalen Gruppen ausgewählt aus OH-, NH₂- und/oder SH-Gruppen. Besonders bevorzugt sind Verbindungen mit mindestens einer, bevorzugt mindestens zwei funktionalen Gruppen ausgewählt aus aromatischen OH-, aromatischen NH₂- und/oder aliphatischen SH-Gruppen. Besonders bevorzugt enthalten die Zusammensetzungen mindestens ein Polyol, Polythiol, Polyamin oder Mischungen daraus, insbesondere aromatische Polyole, aromatische Polyamine und/oder aliphatische Polythiole. "aromatisch" bezeichnet dabei, dass beispielsweise die OH-Gruppe direkt an ein aromatisches System gebunden ist, so dass die negative Ladung nach der Deprotonierung der OH-Gruppe durch mesomere Umlagerung stabilisiert werden kann.

Bevorzugte Alkohole sind Verbindungen die mindestens eine OH-Gruppe pro Molekül, vorzugsweise mindestens zwei OH-Gruppen (Polyol) aufweisen. Eine OH-Gruppe ist eine freie OH-Gruppe. Bevorzugte Polyole sind aromatische Polyole, insbesondere Polyphenole oder Novolak-Harze. Novolak-Harze sind Kondensate von Phenolen mit Formaldehyd. Besonders bevorzugt als Polyole sind Phenol-Novolak und/oder Cresol-Novolak.

Bevorzugte Alkohole, insbesondere Polyole, weisen ein Molekulargewicht von größer 100 g/mol auf, vorzugsweise von 100 bis 20000 g/mol, vorzugsweise 200 bis 10000 g/mol, bevorzugt 400 bis 5000 g/mol. Ganz besonders bevorzugt sind Novolak-Harze, insbesondere Phenol-Novolak und/oder Cresol-Novolak, mit einem Molekulargewicht von 200 bis 20000 g/mol, vorzugsweise 300 bis 10000 g/mol, bevorzugt 400 bis 5000 g/mol. Sofern hierin auf Molekulargewichte von polymeren Verbindungen Bezug genommen wird, beziehen sich die Angaben auf das zahlenmittlere Molekulargewicht Mn, sofern nicht anders angegeben. Das Molekulargewicht kann mittels GPC gegen einen Polystyrolstandard bestimmt werden.

Bevorzugte Amine sind Verbindungen die mindestens eine NH₂-Gruppe pro Molekül, vorzugsweise mindestens zwei NH₂-Gruppen (Polyamin) aufweisen. Eine Amin-Gruppe ist eine freie NH₂-Gruppe. Bevorzugte Amine sind aromatische Amine, insbesondere aromatische Polyamine. Bevorzugt als Amin ist Anilin.

Bevorzugte Thiole sind Verbindungen die mindestens eine Thiol-Gruppe pro Molekül, vorzugsweise mindestens zwei Thiol-Gruppen (Polythiol) aufweisen. Eine Thiol-Gruppe ist eine freie SH-Gruppe. Bevorzugte Polythiole weisen 2 bis 10 Thiol-Gruppen, insbesondere 2 bis 6, ganz besonders bevorzugt 2 bis 4, vor allem 3 oder 4 Thiol-Gruppen auf. Bevorzugt kommen Thiole zum Einsatz die selber ein Polyether oder Polyester sind. Besonders bevorzugte Thiole sind einfache Polyester eines bevorzugt tri- oder tetrafunktionellen Alkohols mit 3-Thiolpropansäure.

Beispiele von Polythiolen sind im Folgenden aufgelistet: 1,2-Ethandithiol, 1,3-Propandithiol, 1,2-Propandithiol, 1,4-Butandithiol, 1,3-Butandithiol, 2,3-Butandithiol, 1,5-Pentandithiol, 1,3-Pentandithiol, 1,6-Hexandithiol, 1,3-Dithio-3-methylbutan, Ethylcyclohexyldithiol, Ethylcyclohexyldithiol, Methylcyclohexyldithiol, Methylsubstitutes Dimercaptodiethylsulfid, Dimethylsubstitutes Dimercaptodiethylsulfid, 2,3-Dimercapto-1-propanol, Bis-(4-mercaptomethylphenyl)ether, 2,2'-Thiodiethanthiol, Pentaerythritol tetrakis (3-mercaptobutylate), Dipentendimercaptan, Trimethylopropan-tris-3-mercaptopropionat, Pentaerythritol-tetra-3-mercaptopropionat und ethoxyliertes Trimethylpropane-tris-3-mercaptopropionat. Besonders bevorzugt sind Trimethylopropan-tris-3-mercaptopropionat, Pentaerythritol-tetra-3-mercaptopropionat und ethoxyliertes Trimethylpropane-tris-3-mercaptopropionat.

Besonders bevorzugt sind Verbindungen mit mindestens zwei funktionalen-Gruppen ausgewählt aus OH- und/oder SH-Gruppen, insbesondere mit einem pKs-Wert von größer 5, insbesondere von 5 bis 20, bevorzugt von 8 bis 14.

Die thermisch expandierbaren Zusammensetzungen enthalten diese mindestens eine Verbindung mit mindestens einer funktionalen Gruppe bevorzugt in einer Menge von 10 bis 80 Gew.-%, vorzugsweise 15 bis 70 Gew.-%, besonders bevorzugt 20 bis 60 Gew.-%, noch weiter bevorzugt 25 bis 50 Gew.-%. Dabei beziehen sich die Gew.-%, soweit nicht anders beschrieben, auf das Gesamtgewicht der Gesamtzusammensetzung vor der Expansion.

In einer anderen bevorzugten Ausführungsform enthalten die thermisch expandierbaren Zusammensetzungen diese mindestens eine Verbindung mit mindestens einer funktionalen Gruppe bevorzugt in einer Menge von 10 bis 80 Gew.-%, vorzugsweise 15 bis 70 Gew.-%, besonders bevorzugt 20 bis 60 Gew.-%, noch weiter bevorzugt 25 bis 50 Gew.-%, jeweils bezogen auf das Bindemittelsystem (Gesamtgewicht der Gesamtzusammensetzung vor der Expansion ohne Füllstoffe).

Als weiteren Bestandteil umfasst die thermisch expandierbare Zusammensetzung mindestens eine Base. Durch die Base wird die Schäumung der expandierbaren Zusammensetzung ermöglicht. Dabei ist der angenommen Mechanismus, dass durch die Base eine Deprotonierung/Aktivierung der Verbindung mit einer funktionalen Gruppe erfolgt und wonach dieses durch einen nucleophilen Angriff des Carbonat zur Freisetzung von CO₂ führt. Die Base sollte somit vorzugsweise geeignet sein, die Verbindung mit mindestens einer funktionalen Gruppe zu deprotonieren bzw. zu aktivieren, insbesondere bei Erhöhung der Temperatur.

Als Base können sowohl anorganische als auch organische Basen eingesetzt werden. In einer bevorzugten Ausführungsform enthält die Zusammensetzung mindestens eine anorganische Base. Geeignete anorganische Basen sind zum Beispiel Hydride oder Hydroxide, insbesondere von Alkali- und/oder Erdalkalimetallen. Besonders bevorzugt sind Natrium- oder Lithium-Hydride oder Hydroxide, insbesondere Natriumhydrid oder Natriumhydroxid.

In einer anderen bevorzugten Ausführungsform enthält die Zusammensetzung mindestens eine organische Base. Dabei ist bevorzugt, dass mindestens eine Stickstoff-haltige Base enthalten ist. Die Stickstoff-haltige Base kann eine ionische oder nicht-ionisch Verbindung sein.

Bevorzugte ionische Basen sind Derivate von Imidazoliumverbindungen, insbesondere 1,3-substituierte Imidazoliumverbindungen. Bevorzugte Verbindungen sind 1-Ethyl-3-Methyl-1H-Imidazoliumacetat, 1-Ethyl-3-Methyl-1H-Imidazoliumthiocyanat, 1-Ethyl-3-Methyl-1H-Imidazoliumcyanocyanamid, 1-Ethyl-3-Methyl-1H-Imidazoliumdiethylphosphat und 1,3-bis(2,6-Düsopropylphenyl)-1H-Imidazolidiniumchlorid.

Bevorzugte nicht-ionische Basen sind nichtionische, Stickstoff-haltige Basen, die mindestens ein tertiäres Stickstoffatom und/oder ein Imin-Stickstoffatom enthalten.

Der Begriff "tertiär", wie hierin verwendet, gibt an, dass an das Stickstoffatom, das in der mindestens einen Base enthalten ist, drei organische Reste kovalent über Einfachbindungen gebunden sind.

Alternativ kann die mindestens eine Base ein Imin-Stickstoffatom enthalten. Der Begriff "Imine", wie hierhin verwendet, bezieht sich auf die bekannte Stoffklasse, und gibt an, dass das Stickstoffatom eine kovalente Doppelbindung zu einem organischen Rest und eine kovalente Einfachbindung zu einem weiteren organischen Rest aufweist. Imine sind Schiff'sche Basen.

Die Klebstoffzusammensetzung kann mehrere der vorstehend beschriebenen nichtionische Basen enthalten, beispielsweise eine Base mit einem Imin-Stickstoff und eine Base mit einem tertiären Stickstoffatom. Die nichtionische Base kann auch sowohl ein tertiäres Amin als auch ein Imin sein, indem sie sowohl ein tertiäres Stickstoffatom als auch ein Imin-Stickstoff enthält.

In bevorzugten Ausführungsformen ist die mindestens eine nichtionische Base ein tertiäres Amin der Formel (BI) NR₃R₄R₅ und/oder ein Imin der Formel (BII) N(=R₆)R₇.

Die Reste R₃ bis R₅ und R₇ werden jeweils unabhängig voneinander ausgewählt aus der Gruppe bestehend aus substituiertem oder unsubstituiertem, linearem oder verzweigtem Alkyl mit 1 bis 20 Kohlenstoffatomen, substituiertem oder unsubstituiertem, linearem oder verzweigtem Alkenyl mit 3 bis 20 Kohlenstoffatomen und substituiertem oder unsubstituiertem Aryl mit 5 bis 20 Kohlenstoffatomen, oder mindestens zwei von R₃ bis R₅ bilden gemeinsam mit dem Stickstoffatom, an das sie gebunden sind, einen 5- bis 10-gliedrigen, heteroalicyclischen Ring oder Heteroaryl-Ring, der optional ein oder mehrere weitere Stickstoffatome, insbesondere 1 weiteres Stickstoffatom, enthält.

R₆ ist ein substituiertes oder unsubstituiertes, lineares oder verzweigtes Alkylenyl mit 3 bis 20 Kohlenstoffatomen oder R₃ und R₄ bilden gemeinsam mit dem Stickstoffatom, an das sie gebunden sind, einen 5- bis 10-gliedrigen, heteroalicyclischen Ring oder Heteroaryl-Ring, der optional weitere Stickstoffatome enthält..

"Alkylenyl", wie hierin verwendet, bezieht sich auf einen Alkylrest, der über eine Doppelbindung an das Stickstoffatom gebunden ist. Falls substituiert, sind die Substituenten wie oben für Alkylreste beschrieben definiert.

In verschiedenen Ausführungsformen der Erfindung sind die tertiären Aminbasen bzw. die Iminbasen, cyclische Verbindungen, die mindestens zwei Stickstoffatome enthalten, d.h. mindestens zwei der Reste R₃ bis R₇ kombinieren miteinander um mit dem Stickstoffatom an das sie gebunden sind, einen Ring zu bilden, und enthalten ferner ein weiteres Stickstoffatom in Form eines Restes -NRR', wobei das Stickstoffatom ein Ringatom ist und der Rest R oder R' an der Ringbildung beteiligt ist. Besonders bevorzugt sind Basen auf Basis von Imidazol oder Imidazolidin. In verschiedenen Ausführungsformen sind die Basen daher beispielsweise Imidazolderivate, wie beispielsweise 1-Alkyl-Imidazol oder 2,4-Dialkylimidazol.

In verschiedenen Ausführungsformen ist die mindestens eine nichtionische Base ausgewählt aus der Gruppe bestehend aus 1-Methylimidazol, 2,4-Ethylmethylimidazol, 1,4-Diazabicyclo[2.2.2]octan (DABCO), 1,8-Diazabicyclo[5.4.0]undec-7-en (DBU) und deren Mischungen.

Bevorzugte Basen sind im Folgenden dargestellt.

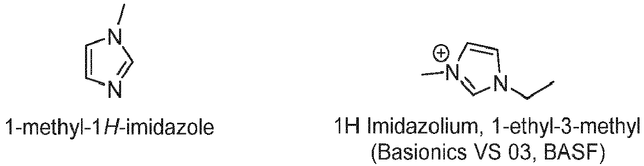

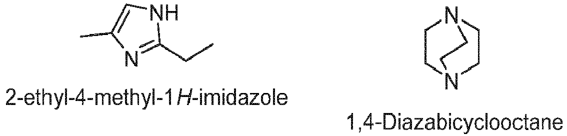

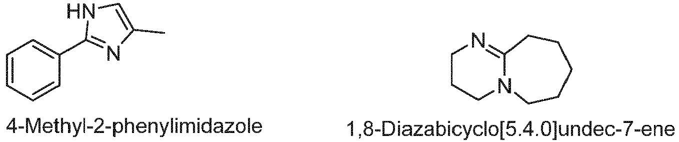

Besonders bevorzugt wird die Base ausgewählt aus der Gruppe bestehend aus 1-Methylimidazol, 2,4-Ethylmethylimidazol, 1-Ethyl-3-methylimidazol und 4-Methyl-2-phenylimidazole.

In besonders bevorzugten Ausführungsformen ist die Base eine nichtionische Stickstoff-haltige Base, insbesondere ausgewählt aus der Gruppe bestehend aus 1-Methylimidazol, 2,4-Ethylmethylimidazol (EMI), 1,4-Diazabicyclo[2.2.2]octan (DABCO), 1,8-Diazabicyclo[5.4.0]undec-7-en (DBU), 1,5-Diazobicyclo[3.4.0]non-5-en (DBN) und Mischungen davon. Vorzugsweise ist die Base ausgewählt aus der Gruppe bestehend aus EMI, DBU und Mischungen davon.

In einer weiteren bevorzugten Ausführungsform ist als Base ein Harnstoff oder ein Harnstoff-Derivat enthalten. Besonders bevorzugt enthält die Zusammensetzung einen substituierten Harnstoff. "Substituiert" in diesem Zusammenhang bedeutet vorzugsweise, dass der Harnstoff an den Stickstoffatomen ein oder mehrere Substituenten ausgewählt aus der Gruppe bestehend aus substituiertem oder unsubstituiertem, linearem oder verzweigtem Alkyl mit 1 bis 20 Kohlenstoffatomen, substituiertem oder unsubstituiertem, linearem oder verzweigtem Alkenyl mit 3 bis 20 Kohlenstoffatomen und substituiertem oder unsubstituiertem Aryl mit 5 bis 20 Kohlenstoffatomen aufweist. Bevorzugte Beispiele sind 1,1-Dimethylharnstoff, p-Chlorphenyl-N,N-dimethylharnstoff (Monuron), 3-Phenyl-1,1-dimethylharnstoff (Fenuron) oder 3,4-Dichlorphenyl-N,N-dimethylharnstoff (Diuron). Ein Harnstoff, insbesondere ein substituierter Harnstoff ist insbesondere bevorzugt, da angenommen wird, dass der Harnstoff die Carbonate zusätzlich durch einen eigenen Mechanismus öffnen kann, wobei zudem Polyurethangruppen entstehen können, welche sich vorteilhaft auf aus Bindemittelsystem auswirken können.

In einer bevorzugten Ausführungsform enthalten die Zusammensetzungen, jeweils bezogen auf das Bindemittelsystem, insbesondere jeweils bezogen auf das Gesamtgewicht, 0,01 bis 10 Gew.%, vorzugsweise 0,1 bis 5 Gew.%, bevorzugt 0,1 bis 2 Gew.% der mindestens einen Base. In einer anderen bevorzugten Ausführungsform enthalten die Zusammensetzungen, bezogen auf das Gesamtgewicht, 0,01 bis 10 Gew.%, vorzugsweise 0,5 bis 7 Gew.%, bevorzugt 1 bis 5 Gew.% mindestens eines Harnstoffs oder eines Harnstoff-Derivats.

In einer bevorzugten Ausführungsform enthält die thermisch expandierbare Zusammensetzung
(1) mindestens einer organischen Verbindung umfassend mindestens zwei cyclische Carbonatgruppen, insbesondere mindestens einer organischen Verbindung der Formel (III) oder (IV);
(2) mindestens einer Verbindung mit mindestens einer funktionalen Gruppe, insbesondere mit mindestens zwei funktionalen Gruppen ausgewählt aus der Gruppe von OH-, NH₂- und/oder SH-Gruppen, insbesondere mindestens eine Verbindung ausgewählt aus Polyolen, Polythiolen und/oder Polyaminen; und
(3) mindestens einen Harnstoff und/oder ein Harnstoff-Derivat.

In einer anderen bevorzugten Ausführungsform enthält die thermisch expandierbare Zusammensetzung
(1) mindestens einer organischen Verbindung umfassend mindestens zwei cyclische Carbonatgruppen, insbesondere mindestens einer organischen Verbindung der Formel (III) oder (IV);
(2) optional mindestens ein weiteres Epoxid
(3) mindestens eine Verbindungen mit mindestens zwei funktionalen-Gruppen ausgewählt aus OH- und/oder SH-Gruppen, insbesondere mit einem pKS-Wert von größer 3, vorzugsweise größer 5, insbesondere von 5 bis 20, bevorzugt von 8 bis 14; und
(4) mindestens eine Base.

In einer weiteren bevorzugten Ausführungsform enthält die thermisch expandierbare Zusammensetzung, jeweils bezogen auf das Bindemittelsystem, insbesondere jeweils bezogen auf das Gesamtgewicht der Zusammensetzung vor der Expansion:
(1) mindestens 20 Gew.-%, insbesondere mindestens 25 Gew.-%, bzw. vorzugsweise 20 bis 90 Gew.-%, insbesondere 25 bis 80 Gew.-%, besonders bevorzugt 25 bis 75 Gew.-%, noch weiter bevorzugt 25 bis 70 Gew.-% und am bevorzugtesten 30 bis 65 Gew.-%. mindestens einer organischen Verbindung umfassend mindestens zwei cyclische Carbonatgruppen, insbesondere mindestens einer organischen Verbindung der Formel (III) oder (IV);
(2) 10 bis 80 Gew.-%, vorzugsweise 15 bis 70 Gew.-%, besonders bevorzugt 20 bis 60 Gew.-%, noch weiter bevorzugt 25 bis 50 Gew.-% mindestens einer Verbindung mit mindestens einer funktionalen Gruppen mit einem pKs-Wert von größer 3, vorzugsweise mindestens eines Polyols, Polythiols, Polyamins oder Mischungen daraus; und
(3) 0,01 bis 10 Gew.%, vorzugsweise 0,1 bis 5 Gew.%, bevorzugt 0,1 bis 2 Gew.% mindestens einer Base, vorzugsweise einer stickstoffhaltigen Base.

Neben den oben genannten Bestandteilen können die thermisch expandierbaren Massen noch weitere übliche Komponenten, wie beispielsweise Füllstoffe, Weichmacher, Reaktivverdünner, Rheologie-Hilfsmittel, Netzmittel, Haftvermittler, Alterungsschutzmittel, Stabilisatoren und/oder Farbpigmente enthalten.

Als Füllstoffe kommen beispielsweise die diversen gemahlenen oder gefällten Kreiden, Calcium-Magnesiumcarbonate, Talkum, Graphit, Schwerspat, Kieselsäuren oder Silica sowie insbesondere silikatische Füllstoffe, wie beispielsweise Glimmer, etwa in Form von Chlorit, oder silikatische Füllstoffe vom Typ des Aluminium-Magnesium-Calcium-Silicats, z. B. Wollastonit, in Frage. Talkum ist ein besonders bevorzugter Füllstoff. Bevorzugt sind die Füllstoffe beschichtet, vorzugsweise mit Stearinsäure oder Stearate. Dadurch wird das Rieselverhalten positiv beeinflusst.

Die Füllstoffe werden vorzugsweise in einer Menge von 0 bis 60 Gew.-%, insbesondere von 0 bis 50 Gew.-%, bevorzugt 0,1 bis 40 Gew.-%, besonders bevorzugt 1 bis 30 Gew.-% jeweils bezogen auf die Masse der gesamten thermisch expandierbaren Zusammensetzung eingesetzt.

Farbgebende Komponenten, insbesondere schwarze Farbstoffe auf Basis von Graphit und/oder Ruß, sind in den erfindungsgemäßen thermisch expandierbaren Zusammensetzungen vorzugsweise in einer Menge von 0 bis 2 Gew.-%, insbesondere von 0,1 bis 0,8 Gew.-%, ganz besonders bevorzugt 0,15 bis 0,4 Gew.-%, jeweils bezogen auf die Masse der gesamten thermisch expandierbaren Zusammensetzung enthalten.

Als Antioxidantien oder Stabilisatoren können beispielsweise sterisch gehinderte Phenole und/oder sterisch gehinderte Thioether und/oder sterisch gehinderte aromatische Amine eingesetzt werden, wie beispielsweise Bis-(3,3-bis-(4'-hydroxy-3-tert. butylphenyl) butansäure)-glykolester oder auch 4-Methylphenol, Reaktionsprodukt mit Dicyclopentadien und Isobutylen (Wingstay L)

Antioxidantien oder Stabilisatoren sind in den erfindungsgemäßen thermisch expandierbaren Zusammensetzungen vorzugsweise in einer Menge von 0 bis 0,5 Gew.-%, insbesondere von 0,1 bis 0,3 Gew.-%, jeweils bezogen auf die Masse der gesamten thermisch expandierbaren Zusammensetzung enthalten.

Reaktivverdünner für Epoxidharze sind Epoxygruppen enthaltende, niederviskose Substanzen (Glycidylether oder Glycidylester) mit aliphatischer oder aromatischer Struktur. Diese Reaktivverdünner können einerseits zur Viskositätserniedrigung des Bindemittel-Systems oberhalb des Erweichungspunktes dienen, andererseits können sie den Vorgelierungsprozess im Spritzguss steuern. Typische Beispiele für geeignete Reaktivverdünner sind Mono-, Di- oder Triglycidylether von C6- bis C14- Monoalkoholen oder Alkylphenolen sowie die Monoglycidylether des Cashewnuss-Schalenöls, Diglycidylether des Ethylenglycols, Diethylenglycols, Triethylenglycols, Tetraethylenglycols, Propylenglycols, Dipropylenglycols, Tripropylenglycols, Tetrapropylenglycols, 1 ,4-Butylenglycols, 1 ,5-Pentandiols, 1 ,6-Hexandiols, Cyclohexandimethanols, Triglycidylether des Trimethylolpropans sowie die Glycidylester von C6- bis C24- Carbonsäuren oder deren Mischungen.

Die erfindungsgemäßen thermisch expandierbaren Zusammensetzungen sind vorzugsweise derart formuliert, dass sie bei 22°C fest sind. Eine thermisch expandierbare Zusammensetzung wird erfindungsgemäß als "fest" bezeichnet, wenn die Geometrie dieser Zusammensetzung sich bei der angegebenen Temperatur innerhalb von 1 Stunde, insbesondere innerhalb von 24 Stunden, nicht unter dem Einfluss der Schwerkraft verformt.

Die erfindungsgemäßen thermisch expandierbaren Zusammensetzungen können durch Mischung der ausgewählten Komponenten in einem beliebigen, geeigneten Mischer, wie beispielsweise einem Kneter, einem Doppel-Z-Kneter, einem Innenmischer, einem Doppelschneckenmischer, einem kontinuierlichen Mischer oder einem Extruder, insbesondere einem Doppelschneckenextruder, hergestellt werden.

Obwohl es vorteilhaft sein kann die Komponenten etwas zu erhitzen, um die Erreichung einer homogenen, einheitlichen Masse zu erleichtern, muss dafür Sorge getragen werden, dass keine Temperaturen erreicht werden, die eine Aktivierung der Härtung bzw. des Aufschäumens bewirken. Die resultierende thermisch expandierbare Zusammensetzung kann unmittelbar nach ihrer Herstellung in Form gebracht werden, beispielsweise durch Blasformen, Pelletierung, Spritzgussverfahren, Formpressverfahren, Stanzverfahren oder Extrusion.

Die Expansion der thermisch expandierbaren Zusammensetzung erfolgt durch Erhitzung, wobei die Zusammensetzung für eine bestimmte Zeit auf eine bestimmte Temperatur erhitzt wird, die ausreichend ist, um die Aktivierung des Carbonats zu bewirken. Die Expansion erfolgt dabei Freisetzung von CO₂ aus den cyclischen Carbonaten. In Abhängigkeit von der Zusammensetzung der expandierbaren Formulierung und den Bedingungen der Fertigungslinie liegen derartige Temperaturen üblicherweise im Bereich von 110 bis 240°C, vorzugsweise 120 bis 210°C, mit einer Verweilzeit von 10 bis 90 Minuten, vorzugsweise von 5 bis 60 Minuten.

Im Bereich des Fahrzeugbaus ist es besonders vorteilhaft, wenn die Expansion der erfindungsgemäßen Zusammensetzungen während der Passage des Fahrzeugs durch den Ofen zur Aushärtung der kathodischen Tauchlackierung erfolgt, so dass auf einen separaten Erhitzungsschritt verzichtet werden kann.

Die thermisch expandierbaren Zusammensetzungen der vorliegenden Erfindung können in einem weiten Bereich von Stütz-, Füll-, Dichtungs- und Klebstoffapplikationen zum Einsatz kommen, beispielsweise im Bereich der Schottteile zur Abdichtung von Hohlräumen in Fahrzeugen oder als Struktur-versteifende Formteile. Aber auch ein Einsatz als Unterfütterungsklebstoff, beispielsweise im Tür oder Dachbereich ist denkbar. Für einen derartigen Einsatzzweck können die erfindungsgemäßen thermisch expandierbaren Zusammensetzungen mittels Direktextrusion appliziert werden. Die Zusammensetzungen können aber auch in extrudierter Form an den Anwendungsort gebracht werden und dort durch Erwärmen des Stahls aufgepresst und angeschmolzen werden. Als dritte Alternative ist auch das Aufbringen als Co-Extrudat denkbar. Bei dieser Ausführungsform wird erfindungsgemäß unter das eigentliche nicht klebrige Formteil aus der erfindungsgemäßen thermisch expandierbaren Zusammensetzung eine zweite klebrige Zusammensetzung in einer dünnen Schicht aufgebracht. Diese zweite klebrige Schicht dient im Rahmen dieser Ausführungsform dazu, das Formteil im Rohbau zu fixieren.

Die thermisch expandierbaren Zusammensetzungen sind dementsprechend besonders für die Herstellung von Formkörpern, insbesondere Schotteilen zur Hohlraumabdichtung und/oder Struktur-versteifende Formteile geeignet, d.h. zur Herstellung von Teilen, die in die Hohlräume von Fahrzeugen eingesetzt werden, anschließend durch Erhitzung expandieren und gleichzeitig aushärten und auf diese Weise den Hohlraum möglichst vollständig abdichten und/oder verstärken.

In einem weiteren Aspekt richtet sich die vorliegende Erfindung dementsprechend auf einen Formkörper, der eine erfindungsgemäße thermisch expandierbare Zusammensetzung aufweist. Dabei kann es sich beispielsweise um ein Schottteil zum Abdichten von Hohlräumen eines Bauteils, das eine Form aufweist, die an den Hohlraum angepasst ist, oder um eine Strukturverstärkungsteil handeln.

Unter einer "Form, die an den Hohlraum angepasst ist" werden dabei erfindungsgemäß alle Geometrien von Schottteilen verstanden, die nach der Expansion eine vollständige Abdichtung des Hohlraums sicherstellen. Dabei kann die Form des Schottteils individuell der Form des Hohlraums nachempfunden sein und entsprechende Spitzen und/oder Rundungen aufweisen; im Falle der erfindungsgemäßen thermisch expandierbaren Zusammensetzungen mit hohen Expansionsgraden, kann aber auch das Einbringen einer entsprechend großen Menge in variabler Form, beispielsweise in Form einer Raupe oder eines abgelängten Strangs des Materials, in den Hohlraum ausreichen, um nach der Expansion eine vollständige Abdichtung des Hohlraums zu gewährleisten.

Derartige Schottteile werden aus den erfindungsgemäßen thermisch expandierbaren Zusammensetzungen üblicherweise durch Spritzgusstechniken hergestellt. Dabei werden die thermisch expandierbaren Zusammensetzungen auf Temperaturen im Bereich von 70 bis 120°C erhitzt und dann in eine entsprechend ausgebildete Form injiziert.

Die erfindungsgemäßen Formkörper können in allen Produkten zum Einsatz kommen, die Hohlräume aufweisen. Dies sind neben den Fahrzeugen beispielsweise auch Flugzeuge, Schienenfahrzeuge, Haushaltsgeräte, Möbel, Gebäude, Wände, Abtrennungen oder auch Boote.

Ein weiterer Gegenstand der vorliegenden Erfindung ist ein Verfahren zum Abdichten und Füllen von Hohlräumen in Bauteilen, zum Verstärken oder Versteifen von Bauteilen, insbesondere hohlen Bauteilen, und zum Verkleben von beweglichen Bauteilen unter Verwendung der hierin beschriebenen Zusammensetzungen und Formkörper. Vorzugsweise ist das Verfahren ein Verfahren zur Abdichtung von Hohlräumen eines Bauteils, wobei ein erfindungsgemäßes Schottteil in den Hohlraum eingebracht wird und anschließend auf eine Temperatur oberhalb von 30°, vorzugsweise von 50°C und 250°C, besonders bevorzugt 80°C und 160°C erhitzt wird, so dass die thermisch expandierbare Zusammensetzung expandiert und den Hohlraum abdichtet, füllt oder verstärkt.

Ein weiterer Gegenstand der vorliegenden Erfindung ist die Verwendung eines erfindungsgemäßen Formkörpers oder Schottteils zur akustischen Abdichtung von Hohlräumen in Bauteilen und/oder zur Abdichtung von Hohlräumen in Bauteilen gegen Wasser und/oder Feuchtigkeit.

Noch ein weiterer Gegenstand der vorliegenden Erfindung ist die Verwendung eines erfindungsgemäßen Formkörpers zum Verstärken oder Versteifen von Bauteilen, insbesondere hohlen Bauteilen.

Die nachfolgenden Beispiele sollen die Erfindung näher erläutern, wobei die Auswahl der Beispiele keine Beschränkung des Umfanges des Erfindungsgegenstandes darstellen soll. Bei den Zusammensetzungen sind alle Mengenangaben Gewichtsteile, wenn nicht anders angegeben.

### Ausführungsbeispiele

### Synthese von eines organisches Carbonats aus Epoxid und Kohlendioxid

Tetrabutylammoniumiodid wurde von Acros erhalten. Die Epoxide D.E.R. 331 und D.E.R.749 wurden von Dow Chemicals erhalten.

Das Carbonat wurde unter Verwendung eines zweiteiligen 1 Liter Glasreaktors ausgestattet mit einem hohlen mechanischen Rührer und einem Thermometer hergestellt. Die Temperatur wurde durch das Verbinden des Thermometers mit einer Heizplatte konstant gehalten. Trockeneis (CO₂) wurde in einen anderen 1 Liter Rundkolben gegeben. Das freiwerdende Gas wurde durch einen Polyethylenschlauch über den hohlen Rührer in den Hauptreaktor eingeleitet. Das Epoxid wurde mit dem entsprechenden Katalysator (10 Gew.-% des Epoxids) in den Reaktor gegeben und unter ständigem Rühren bei 140°C erhitzt. In der Epoxidmischung waren Blasen zu beobachten.

Der 1 Liter Rundkolben wurde alle 4-5 h mit Trockeneis aufgefüllt. Nach 2-3 Tagen wurde die Mischung weiß und hochviskos. Zu diesem Zeitpunkt wurde die Reaktionsmischung abgekühlt. Als Produkt konnten die Carbonate von D.E.R. 331 (Produkt aus Epichlorohydrin mit Bisphenol A) und D.E.R. 749 (Produkt aus Epichlorohydrin und Tetramethylolmethan) erhalten werden.

Die Synthese eines organischen Carbonats ausgehend von polyfunktionellen Epoxids unter Verwendung von Tetrabutylammoniumiodid als Synthesekatalysator wird beispielhaft in folgendem Schema gezeigt:

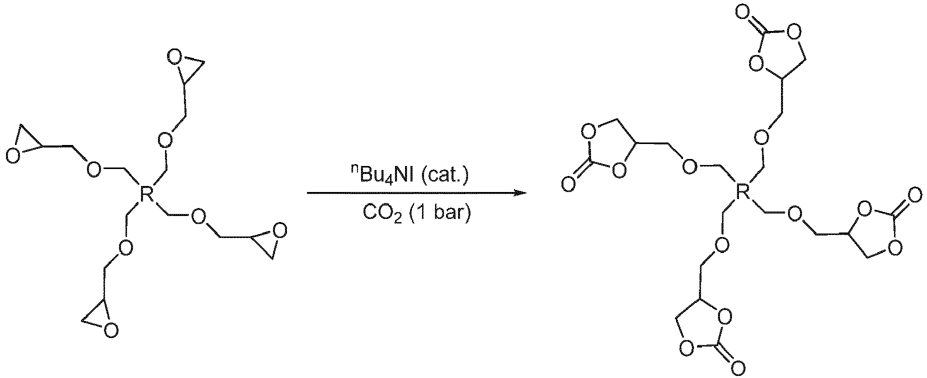

### Expandierbare Zusammensetzungen

Die expandierbaren Zusammensetzungen wurden durch Vermischen der in der nachstehenden Tabelle genannten Bestandteile (in Gewichtsteilen) erhalten, wobei die Base als letztes hinzugefügt wurde. Die Expansion und Härtung erfolgte anschließend bei 180 °C für 60 min.

| Beispiel | 1 | 2 | 3 | 4 | 5 | 6 | 7 |
|---|---|---|---|---|---|---|---|
| DER 331 - Carbonat | 33,6 | 54,81 | 57 | 57 | | 33 | |
| DER 749 - Carbonat | | | | | 14,0 | | 33 |
| DER 331 | 8,4 | | | | 23,6 | 22 | 22 |
| TMPMP | 30 | 30 | 42 | 42 | | | |
| Phenol-Novolak | | | | | 24,2 | 27,5 | 27,5 |
| Cab-o-Sil TS 720 (pyrogene Kieselsäure) | 2,5 | 2,5 | | | 2,0 | 2,5 | 2,5 |
| Bariumsulfat | 24,5 | 11,69 | | | 15,0 | 11,7 | 11,7 |
| Monarch 580 (Ruß) | | | | | 1,0 | | |
| Glasfasern (∼2mm lang) | | | | | 10,0 | | |
| Glashohlkugeln VS5500 | | | | | 7,0 | | |
| | | | | | | | |
| 2-Ethyl-4-methylimidazol | 1 | 1 | | | | | |
| Natriumhydrid (35%ig auf Mineralöl) | | | 1 | | | | |
| Natriumhydroxid (feingemalen) | | | | 1 | | | |
| 1,1-Dimethylharnstoff | | | | | 3,2 | 3,3 | 3,3 |

| | | | | | | | |
|---|---|---|---|---|---|---|---|
| TMPMP: Trimethylolpropantris(3-mercaptopropionat) | | | | | | | |

Die so erhaltenen Schäume zeichnen sich durch eine hohe, erreichbare Expansionsrate aus. Ferner zeichnen sich die Schäume basierend auf cyclischen organischen Carbonaten durch eine sehr saubere Zersetzung aus. Es konnten keine Zersetzungsprodukte aus dem Carbonat detektiert werden.

## Patentansprüche

1. Thermisch expandierbare Zusammensetzung, enthaltend
(1) mindestens eine organische Verbindung umfassend mindestens zwei cyclische Carbonatgruppen,
(2) mindestens eine Verbindung mit mindestens einer funktionalen Gruppen mit einem pKs-Wert von größer 3, und
(3) mindestens eine Base.

2. Thermisch expandierbare Zusammensetzung nach Anspruch 1, **dadurch gekennzeichnet, dass** die organische Verbindung ein Polyether und/oder Polyester, insbesondere Polyether ist, an welchen mindestens zwei cyclische Carbonatgruppen gebunden sind.

3. Thermisch expandierbare Zusammensetzung nach Anspruch 1 oder 2, **dadurch gekennzeichnet, dass** die mindestens eine organische Verbindung ein cyclisches organisches Carbonat der Formel (I) 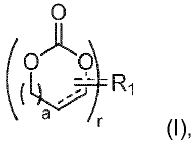 ist, wobei
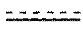 eine Einfach- oder Doppelbindung, vorzugsweise eine Einfachbindung ist, wobei wenn der Ring eine Doppelbindung enthält, R₁ nicht über eine exo-Doppelbindung sondern über eine Einfachbindung gebunden ist und umgekehrt, und R₁ lineares oder verzweigtes, substituiertes oder unsubstituiertes Alkyl, lineares oder verzweigtes, substituiertes oder unsubstituiertes Heteroalkyl, lineares oder verzweigtes, substituiertes oder unsubstituiertes Alkenyl, lineares oder verzweigtes, substituiertes oder unsubstituiertes Alkinyl, substituiertes oder unsubstituiertes Cycloalkyl, substituiertes oder unsubstituiertes Aryl, oder -C(O)-R^{a}, wobei R^{a} H, lineares oder verzweigtes, substituiertes oder unsubstituiertes Alkyl, lineares oder verzweigtes, substituiertes oder unsubstituiertes Heteroalkyl, lineares oder verzweigtes, substituiertes oder unsubstituiertes Alkenyl, lineares oder verzweigtes, substituiertes oder unsubstituiertes Alkinyl, substituiertes oder unsubstituiertes Cycloalkyl, substituiertes oder unsubstituiertes Aryl ist, ist, vorzugsweise ein lineares oder verzweigtes, substituiertes oder unsubstituiertes Heteroalkyl,
a eine ganze Zahl von 0 bis 5, vorzugsweise 0 oder 1 und besonders bevorzugt 0 ist und
r eine natürliche Zahl von 1 bis 10, vorzugsweise von 1 bis 4 und besonders bevorzugt 2 bis 4 ist.

4. Thermisch expandierbare Zusammensetzung nach einem der Ansprüche 1 bis 3, **dadurch gekennzeichnet, dass** die mindestens eine organische Verbindung ein cyclisches organisches Carbonat der Formel (II) 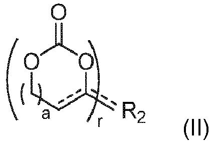 ist, wobei
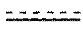 , a und r wie oben definiert sind und R₂ wie R₁ definiert ist.

5. Thermisch expandierbare Zusammensetzung nach einem der Ansprüche 1 bis 4, **dadurch gekennzeichnet, dass** die mindestens eine organische Verbindung ein cyclisches organisches Carbonat der Formel (III) oder (IV) 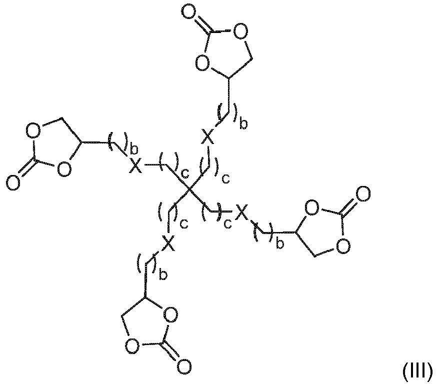 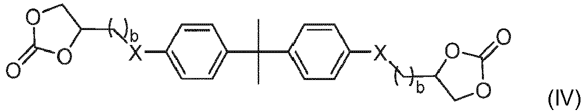 ist, wobei
jedes b und c jeweils unabhängig voneinander eine natürliche Zahl von 1 bis 5 vorzugsweise 1 oder 2 und besonders bevorzugt 1 ist, und
jedes X unabhängig ausgewählt ist aus der Gruppe bestehend aus O, S und N und vorzugsweise jedes X O ist.

6. Thermisch expandierbare Zusammensetzung nach einem der Ansprüche 1 bis 5, **dadurch gekennzeichnet, dass** die mindestens eine organische Verbindung ein cyclisches organisches Carbonat der Formel (V) oder (VI) ist

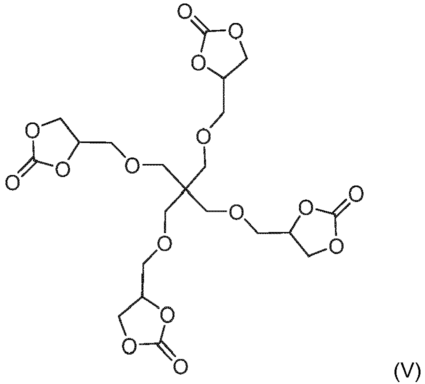

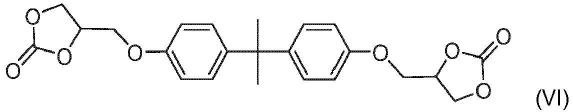

7. Thermisch expandierbare Zusammensetzung nach einem der Ansprüche 1 bis 6, **dadurch gekennzeichnet, dass** die mindestens eine Base eine Stickstoff-haltige Base ist.

8. Thermisch expandierbare Zusammensetzung nach Anspruch 7, **dadurch gekennzeichnet, dass** die mindestens eine Base ausgewählt wird aus der Gruppe bestehend aus 1-Methylimidazol, 2,4-Ethylmethylimidazol (EMI), 1,4-Diazabicyclo[2.2.2]octan (DABCO), 1,8-Diazabicyclo[5.4.0]undec-7-en (DBU), 1,5-Diazobicyclo[3.4.0]non-5-en (DBN) und Mischungen davon.

9. Thermisch expandierbare Zusammensetzung nach einem der Ansprüche 1 bis 8, **dadurch gekennzeichnet, dass** die thermische expandierbare Zusammensetzung bezogen auf das Bindemittelsystem (Gesamtgewicht der Zusammensetzung vor der Expansion ohne Füllstoffe) 20 bis 90 Gew.-%, vorzugsweise 25 bis 80 Gew.-%, besonders bevorzugt 25 bis 75 Gew.-%, noch weiter bevorzugt 25 bis 70 Gew.-% und am bevorzugtesten 30 bis 65 Gew.-% der mindestens einen organischen Verbindung umfassend mindestens eine cyclische Carbonatgruppe umfasst.

10. Thermisch expandierbare Zusammensetzung nach einem der Ansprüche 1 bis 9, **dadurch gekennzeichnet, dass** die mindestens eine funktionalen Gruppe der mindestens einen Verbindung ausgewählt wird aus OH-, NH₂- und/oder SH-Gruppen, insbesondere aus aus aromatischen OH-, aromatischen NH₂- und/oder aliphatischen SH-Gruppen.

11. Thermisch expandierbare Zusammensetzung nach einem der Ansprüche 1 bis 10, **dadurch gekennzeichnet, dass** die mindestens eine Verbindungen mindestens zwei funktionalen-Gruppen ausgewählt aus OH- und/oder SH-Gruppen aufweist, insbesondere mit einem pKs-Wert größer 5, insbesondere von 5 bis 20, bevorzugt von 8 bis 14..

12. Formkörper, **dadurch gekennzeichnet, dass** der Formkörper eine thermisch expandierbare Zusammensetzung nach einem der Ansprüche 1 bis 11 aufweist.

13. Verfahren zum Abdichten und Füllen von Hohlräumen in Bauteilen, zum Verstärken oder Versteifen von Bauteilen, insbesondere hohlen Bauteilen, und zum Verkleben von beweglichen Bauteilen unter Verwendung einer thermisch expandierbaren Zusammensetzung nach einem der Ansprüche 1 bis 11 oder einem Formkörper nach Anspruch 12.

14. Verfahren nach Anspruch 13 zum Abdichten und Füllen von Hohlräumen in Bauteilen, zum Verstärken oder Versteifen von Bauteilen, **dadurch gekennzeichnet, dass** ein Formkörper nach Anspruch 11 in ein Bauteil, insbesondere den Hohlraum eines Bauteils, eingebracht wird und anschließend auf eine Temperatur oberhalb von 30°C, vorzugsweise im Bereich von 50°C bis 250°C, besonders bevorzugt 80°C bis 160°C erhitzt wird, so dass die thermisch expandierbare Zusammensetzung expandiert und das Bauteil abdichtet, füllt, verstärkt oder versteift.

15. Verwendung eines Formkörpers nach Anspruch 14 zur akustischen Abdichtung von Hohlräumen in Bauteilen und/oder zur Abdichtung von Hohlräumen in Bauteilen gegen Wasser und/oder Feuchtigkeit oder zum Verstärken oder Versteifen von Bauteilen, insbesondere hohlen Bauteilen.
